# NOUVEAU FASCICULE DE BREVET EUROPEEN

(11) **EP 1 300 167 B2**
(45) Date de publication et mention de la décision concernant l'opposition: **19.03.2014**
(45) Mention de la délivrance du brevet: 08.03.2006
(21) Numéro de dépôt: 02080290.6
(22) Date de dépôt: 24.07.1995
(51) Int. Cl.: A61M 1/16

(54) **Procédé pour déterminer le vieillissement d'un filtre de liquide**
Verfahren zur Bestimmung der Alterung eines Flüssigkeitsfilter
Method for the determination of the ageing of a fluid filter

(30) Priorité: 26.07.1994 FR 9409499
(43) Date de publication de la demande: 09.04.2003
(62) Demande divisionnaire de: 95420216.4
(73) Titulaire: Gambro Industries SAS, 69330 Meyzieu (FR)
(72) Inventeur: Riquier, Jean Claude, 69140 Rillieux (FR); Chevallet, Jaques, 69360 Serezin du Rhône (FR)
(74) Mandataire: Ponzellini, Gianmarco

(56) Documents cités:
- EP-A- 0 571 303
- DE-A1- 3 442 744
- US-A- 4 936 980

## Description

La présente invention a pour objet un procédé de détermination du vieillissement d'un filtre de liquide.

Les patients souffrant d'insuffisance rénale peuvent être soumis à différents traitements impliquant une circulation extracorporelle de sang, l'hémodialyse, l'hémofiltration et l'hémodiafiltration.

L'hémodialyse consiste à faire circuler le sang du patient dans un premier compartiment d'un dialyseur et un liquide de dialyse dans un second compartiment du dialyseur, les deux compartiments étant séparés par une membrane semi-perméable permettant un transfert diffusif de solutés au travers de la membrane, du compartiment où la concentration en un soluté particulier est la plus importante vers le compartiment où la concentration en ce soluté est la moins importante.

L'hémofiltration consiste à extraire du sang du patient une fraction d'eau plasmatique ou filtrat au moyen d'un hémofiltre et à infuser simultanément au patient un liquide de substitution pour compenser, en général seulement partiellement, la quantité de filtrat extraite.

L'hémodiafiltration est une combinaison des deux traitements précités.

Le liquide de dialyse et le liquide de substitution sont des liquides ayant sensiblement la même composition. Ils sont isotoniques au sang dont ils contiennent les principaux électrolytes.

Dans les machines de dialyse classiques, le liquide de dialyse est préparé par mélange d'eau et de solutions concentrées ou de sels pulvérulents, comprenant les principaux électrolytes du sang. Ce liquide de dialyse n'est ni stérile ni apyrogène, c'est-à-dire qu'il peut contenir des micro-organismes vivants (bactéries) ainsi que des éléments, dits pyrogènes, dont l'introduction dans le corps peuvent produire des effets indésirables, tels que fièvre, frissons, nausées ou réactions anaphylactoïdes.

Bien que la membrane du dialyseur isole le sang du liquide de dialyse et que, en cours de traitement, une pression transmembranaire positive soit établie entre le compartiment sang et le compartiment liquide de dialyse du dialyseur pour prévenir le passage de liquide de dialyse dans le sang, toute contamination du sang par des bactéries et des éléments pyrogènes contenus dans le liquide de dialyse n'est pas totalement exclue, notamment dans le cas d'une rupture de filtre ou dans le cas d'une inversion accidentelle du sens de la pression transmembranaire lorsqu'un dialyseur à haute perméabilité hydraulique est utilisé.

Aussi l'intérêt d'utiliser du liquide de dialyse stérile et apyrogène a été exprimé à plusieurs reprises. On a en particulier proposé de filtrer le liquide de dialyse dans un filtre ayant une première et une seconde chambre séparées par une membrane ayant une perméabilité hydraulique élevée, la première chambre ayant une entrée pour l'introduction du liquide à filtrer et une sortie pour l'évacuation des substances retenues par la membrane avec une fraction du liquide introduit dans le filtre (voir "Investigation of the Permeability of Highly Permeable Polysulfone Membranes for Pyrogens" In Contr. Nephrol., vol. 46, pp. 174-183, Karger, Basel 1985).

Le brevet européen 0 270 794 décrit une machine de dialyse dont le circuit de liquide de dialyse comprend :
- une canalisation d'alimentation sur laquelle est disposé un filtre ayant une première et une seconde chambres séparées par une membrane de filtration, la canalisation d'alimentation ayant une première portion reliant une source de liquide de dialyse à une entrée de la première chambre, et une secondé portion ayant une extrémité connectée à une sortie de la seconde chambre du filtre et une autre extrémité connectable à une entrée d'un premier compartiment d'un dialyseur ;
- une canalisation d'évacuation ayant une extrémité connectable à une sortie du premier compartiment du dialyseur ; et
- une canalisation de purge, sur laquelle est disposée une vanne, reliant une sortie de la première chambre du filtre à la canalisation d'évacuation.

En fonctionnement, la vanne disposée sur la canalisation de purge est ouverte à intervalles de temps réguliers pour purger la première chambre du filtre des micro-organismes et éléments pyrogènes retenus par la membrane.

Cette machine présente plusieurs inconvénients. En particulier, il est clair que la vanne de la canalisation de purge ne peut être ouverte fréquemment à cause de la perturbation du traitement qui en résulte. (En effet, compte ténu de la perte de charge importante que représente la membrane du filtre, lorsque la vanne est ouverte, tout le liquide de dialyse provenant de la source de liquide de dialyse s'écoule par la canalisation de purge et le dialyseur n'est plus alimenté en liquide de dialyse frais). Par ailleurs, entre deux ouvertures successives de la vanne, il s'écoule un laps de temps pendant lequel les substances indésirables s'accumulent dans la première chambre du filtre où elles ont tendance à être entraînées par convection vers la membrane et à l'encrasser. Il en résulte que, en cas de rupture de la membrane du filtre, ces substances accumulées sont envoyées dans le dialyseur et la portion de circuit de dialyse située en amont et en aval de celui-ci, qu'elles contaminent. Aussi, le balayage tangentiel de la membrane qui résulte de l'ouverture intermittente de la vanne ne peut pas être suffisamment long pour décoller de la membrane toutes les substances qui y ont adhéré.

Un but de l'invention est de réaliser-un procédé et un dispositif pour détecter le vieillissement d'un filtre de liquide. Pour atteindre ce but, on prévoit conformément à l'invention un procédé selon la revendication 1.

Le document EP 0 571 303 B1 décrit un circuit hydraulique pour rein artificiel comportant un ultrafiltre ayant une première et une seconde chambres séparées par une membrane, une première portion d'une canalisation d'alimentation ayant son autre extrémité connectée à une entrée de la première chambre et une seconde portion de la canalisation d'alimentation ayant son autre extrémité connectée à une sortie de la seconde chambre, des moyens pour déterminer la pression transmembranaire dans l'ultrafiltre, des moyens de commande pour comparer la pression transmembranaire mesurée à au moins une valeur de seuil, de façon que la première et la seconde portions de la canalisation d'alimentation soient reliées directement par des moyens de dérivation et que le passage du liquide soit interdit dans l'ultrafiltre.

Le dispositif comporte des moyens pour mémoriser une pression transmembranaire initiale à la première mise en service du filtre et des moyens pour comparer la pression transmembranaire initiale à la pression transmembranaire mesurée. Le dispositif peut comporter en outre des moyens d'alarme pour émettre une alarme lorsque la pression transmembranaire mesurée s'écarte d'une quantité prédéterminée de la pression transmembranaire initiale.

On a observé que, à débit de liquide de dialyse égal dans le circuit de liquide de dialyse, la pression transmembranaire dans le filtre augmentait au cours du temps, de sorte que la comparaison entre le pression transmembranaire à la mise en service du filtre et la pression transmembranaire mesurée à un instant donné de l'utilisation du filtre donne une idée juste du vieillissement du filtre. On peut donc définir pour chaque filtre une durée d'utilisation individuelle optimale correspondant à une augmentation relative de la pression transmembranaire.

Le dispositif peut comporter des moyens de contrôle d'une quantité de liquide de dialyse, tels qu'un débitmètre, disposés sur la canalisation d'alimentation et le filtre peut être placé sur la canalisation d'alimentation entre la source de liquide de dialyse et les moyens de contrôle d'une quantité de liquide de dialyse.

Cette disposition présente plusieurs avantages : en premier lieu, les moyens de contrôle sont parcourus par un liquide filtré, ce qui est tout à fait approprié lorsque ces moyens sont constitués par un débitmètre, et particulièrement un débitmètre à turbine ou à engrenages dont le fonctionnement peut être sérieusement perturbé par une impureté solide et est en général altéré par un encrassement progressif. Par ailleurs, de tels moyens de contrôle peuvent faire partie d'un système de maîtrise volumétrique de l'ultrafiltration au moyen duquel, dans une portion du circuit de liquide de dialyse comprenant l'échangeur, la quantité de liquide de dialyse qui sort de cette portion de circuit est maintenue égale à la quantité de liquide de dialyse qui y entre. De seconds moyens de contrôle de la quantité de liquide de dialyse sont alors disposés sur la canalisation d'évacuation du circuit de liquide de dialyse. En connectant le filtre au circuit de liquide de dialyse à l'extérieur de la portion du circuit de liquide de dialyse où circule un volume constant de liquide, on évite d'avoir à connecter la canalisation de purge à la canalisation d'évacuation du circuit de dialyse en amont des seconds moyens de contrôle, c'est-à-dire aussi d'avoir à y introduire les impuretés retenues par le filtre, comme c'est le cas dans le dispositif décrit dans le brevet européen 0 270 794 mentionné plus haut. Encore un autre avantage de cette disposition est que la canalisation de purge peut être reliée directement à l'égout par une voie de sortie de la machine de dialyse indépendante de la voie de sortie par laquelle la canalisation d'évacuation de liquide usé est reliée à l'égout. De la sorte, la portion du circuit de liquide de dialyse qui se trouve en amont du filtre est totalement isolée, par le filtre, de la portion de circuit de liquide de dialyse qui se trouve en aval du filtre. La contamination de la canalisation d'évacuation, en aval comme en amont (rétro-contamination) de la jonction de la canalisation de purge à la canalisation d'évacuation, qui se produit sur le dispositif décrit dans l'état de la technique, est donc totalement évitée.

L'invention a aussi pour objet un procédé de détermination du vieillissement d'un filtre ayant deux chambres séparées par une membrane de filtration, une première chambre étant reliée à une source de liquide de traitement et une seconde chambre ayant une sortie pour du liquide de traitement filtré, caractérisé en ce qu'il comprend les étapes de :
- mesurer et mémoriser, pour un débit de liquide de traitement donné à la première mise en service du filtre, une pression transmembranaire de référence dans le filtre à la première mise en service du filtre;
- mesurer, à chaque utilisation ultérieure du filtre ou à intervalles de temps réguliers, la pression transmembranaire, au débit de liquide de traitement donné ;
- comparer la pression transmembranaire mesurée à la pression transmembranaire de référence.

D'autres caractéristiques et avantages de l'invention apparaîtront à la lecture de la description qui va suivre.

On se reportera aux dessins annexés sur lesquels :
La figure 1 est un schéma d'un dispositif de dialyse comprenant un système de filtration de liquide de dialyse ; et
La figure 2 est un schéma d'un dispositif d'hémodiafiltration comprenant un système de filtration de liquide de traitement.

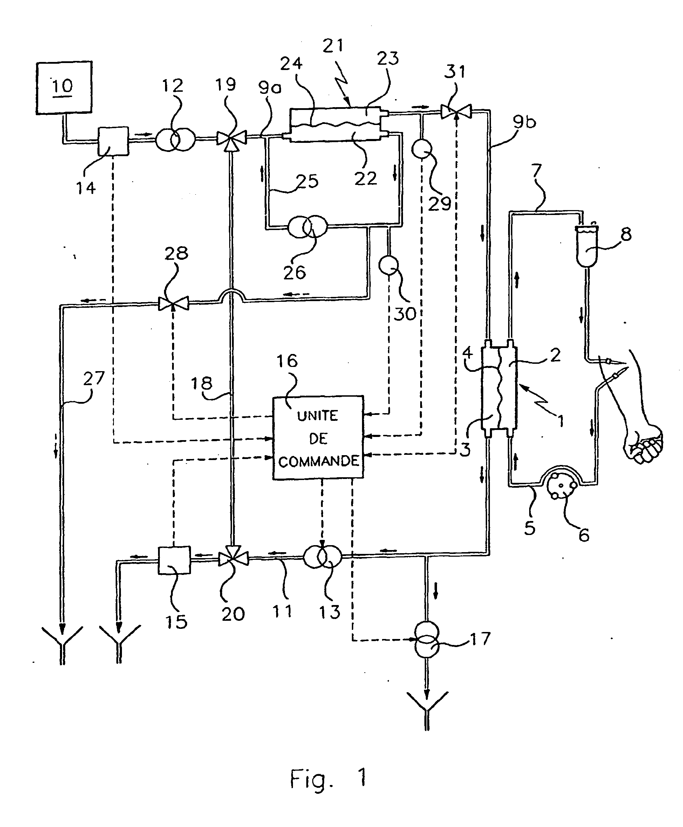

Le dispositif de traitement de sang par circulation extracorporelle représenté sur la figure 1 est propre à effectuer un traitement de dialyse. Ce dispositif comprend un hémodialyseur 1 ayant deux compartiments 2, 3 séparés par une membrane semi-perméable 4, un premier compartiment 2 étant relié à un circuit de circulation extracorporelle de sang, un second compartiment 3 étant relié à un circuit de circulation de liquide de dialyse. De façon classique, tous les composants du dispositif qui va être décrit ci-dessous, à l'exception du circuit de circulation de sang et de l'hémodialyseur, sont agencés au sein d'une machine dite de dialyse.

Le circuit de circulation de sang comprend une canalisation de prélèvement 5 connectée à une entrée du premier compartiment 2, sur laquelle est disposée une pompe 6, et une canalisation de restitution 7 connectée à une sortie du premier compartiment 2, sur laquelle est monté un piège à bulles 8.

Le circuit de liquide de dialyse comprend une canalisation d'alimentation (9a, 9b) en liquide de dialyse frais, reliant une source de liquide de dialyse 10 à une entrée du second compartiment 3 de l'hémodialyseur 1, et une canalisation d'évacuation 11 de liquide usé reliant une sortie du second compartiment 3 de l'hémodialyseur 1 à l'égout.

La source de liquide de dialyse est, par exemple, un générateur de liquide de dialyse 10 au moyen duquel de l'eau est réchauffée, dégazée puis mélangée, dans des proportions déterminées, à des solutions concentrées contenant les principaux électrolytes du sang. Le liquide de dialyse produit n'est ni stérile, ni apyrogène.

Le circuit de liquide de dialyse comprend des moyens de circulation de liquide de dialyse constitués par une première pompe 12 disposée sur la canalisation d'alimentation (9a, 9b) et une deuxième pompe 13 disposée sur la canalisation d'évacuation 11.

Le dispositif comprend aussi un système de maîtrise volumétrique de l'ultrafiltration comprenant un premier moyen de contrôle d'une quantité de liquide de dialyse, tel qu'un premier débitmètre 14, disposé sur la canalisation d'alimentation (9a, 9b) et un second moyen de contrôle d'une quantité de liquide de dialyse, tel qu'un second débitmètre 15, disposé sur la canalisation d'évacuation 11 en aval de la deuxième pompe de circulation 13. Les mesures effectuées par les deux débitmètres sont comparées par une unité de calcul et de commande 16 qui pilote la deuxième pompe 13 de circulation de liquide de dialyse de façon que les débits mesurés par les deux débitmètres soient identiques. Le système de maîtrise de l'ultrafiltration comprend en outre une pompe d'ultrafiltration 17 connectée à la canalisation d'évacuation en amont de la seconde pompe 13. Grâce à l'asservissement de la seconde pompe 13 décrit plus haut, la quantité de liquide extraite du circuit de liquide de dialyse par la pompe d'ultrafiltration 17 correspond exactement à la quantité d'eau plasmatique qui passe du sang dans le liquide de dialyse par ultrafiltration au travers de la membrane 4 sous l'effet de la dépression relative créée dans le circuit de liquide de dialyse par la pompe d'ultrafiltration 17. Une canalisation de dérivation 18 relie la canalisation d'alimentation (9a, 9b) à la canalisation d'évacuation 11 auxquelles elle est connectée, par l'intermédiaire de deux vannes trois voies 19, 20, respectivement en aval du premier débitmètre 14 et en amont du second débitmètre 15. Cette ligne de dérivation 18 permet de disposer les débitmètres 14, 15 directement en série pour un étalonnage programmé à intervalles réguliers.

Le dispositif comprend en outre un filtre 21 pour filtrer le liquide de dialyse produit par le générateur de liquide de dialyse 10. Le filtre 21 a une première et une seconde chambres 22, 23 séparées par une membrane de filtration 24, la première chambre 22 ayant une entrée connectée à une première portion 9a de la canalisation d'alimentation et la seconde chambre 23 ayant une sortie connectée à une seconde portion 9b de la canalisation d'alimentation, sur laquelle est disposé une vanne 31.

Une canalisation de bouclage 25, sur laquelle est disposée une pompe de balayage 26, relie une sortie de la première chambre 22 du filtre 21 à l'entrée de cette première chambre. Une canalisation de purge 27 de la première chambre 22 du filtre 21, sur laquelle est disposé un organe de contrôle de débit tel qu'une vanne 28, est connectée à la canalisation de bouclage 25 entre la sortie de la première chambre 22 du filtre 21 et la pompe 26. La canalisation de purge 27 est reliée à l'égout par une sortie de la machine de dialyse distincte de la sortie qui forme l'extrémité de la canalisation d'évacuation 11 du liquide usé.

Deux capteurs de pression 29, 30 sont disposés respectivement sur la seconde portion 9b de la canalisation d'alimentation et sur la canalisation de bouclage 25, en sortie de la première et de la deuxième chambres 22, 23 du filtre 21, pour mesurer la pression dans ces chambres. Les informations délivrées par les capteurs de pression 29, 30 sont fournies à l'unité de commande et de calcul 16 qui peut calculer la pression transmembranaire dans le filtre 21 et commander le fonctionnement du dispositif en fonction des valeurs mesurées et calculées des pressions dans le filtre 21, comme cela va être expliqué dans ce qui suit.

Lorsque les étapes préparatoires au traitement sont achevées, c'est-à-dire le rinçage et le remplissage initiaux du circuit de liquide de dialyse 9a, 9b, 11, de l'hémodialyseur 1, et du circuit sang 5, 7, 8 et la connexion du circuit sang au circuit vasculaire du patient, le liquide de dialyse produit par le générateur 10 de liquide de dialyse est mis en circulation dans le circuit de liquide de dialyse au moyen des pompes 12 et 13, et le sang du patient est mis en circulation dans le circuit sang au moyen de la pompe 6 (la vanne 31 est alors ouverte et les vannes 19, 20 sont disposées de façon à permettre la circulation dans le canalisation d'alimentation 9a, 9b et dans la canalisation d'évacuation 11).

En outre, la vanne 28 de la canalisation de purge 27 est fermée et la pompe de balayage 26 tourne à une vitesse prédéterminée de sorte que du liquide non filtré provenant du générateur circule en permanence dans la première chambre 22 du filtre 21 et balaye la membrane 24, ce qui a pour effet de s'opposer à son encrassement en maintenant en suspension les impuretés arrêtées par la membrane du filtre.

De façon classique, les débitmètres 14 et 15 sont étalonnés à intervalles de temps réguliers. Lors de ces étalonnages successifs, les vannes 19 et 20 sont disposées de façon que le liquide de dialyse circule dans la canalisation de dérivation 18, et la première pompe de circulation 12 et la pompe d'ultrafiltration 17 tournent à leur valeur de consigne tandis que la deuxième pompe de circulation 13 est stoppée.

La purge de la première chambre 22 du filtre 21, destinée à éliminer les impuretés arrêtées par la membrane 24, est commandée lorsqu'un niveau d'encrassement prédéterminé de la membrane est détecté. Plus précisément, l'unité de commande 16 compare soit en continu, soit à intervalles de temps réguliers, la valeur instantanée de la pression transmembranaire dans le filtre 21, calculée à partir des informations délivrées par les capteurs de pression 29, 30, à une valeur de référence calculée en début de séance. Lorsque la valeur instantanée excède la valeur de référence d'une quantité prédéterminée, une purge de la première chambre 22 du filtre 21 est commandée lors de la phase d'étalonnage des débitmètres 14, 15 subséquente. La vanne 31 est alors fermée et la vanne de purge 28 est ouverte le temps nécessaire à l'évacuation, par la canalisation de purge 27, du liquide contenu dans la première chambre 22 du filtre 21 et dans la canalisation de bouclage 25.

La fréquence des purges est calculée par l'unité de calcul et est comparée à une fréquence de référence. Si la fréquence calculée atteint ou excède la fréquence de référence, la vitesse de rotation de la pompe 26 est augmentée pour que l'efficacité du nettoyage de la membrane, par balayage tangentiel soit accrue.

Selon l'invention, on mesure le vieillissement du filtre 21 en partant de l'observation qu'au cours du temps et en dépit du nettoyage continu auquel la membrane 24 est soumise, la pression transmembranaire dans le filtre augmente, à débits de liquide de dialyse égaux. L'unité de commande et de calcul 16 compare donc à intervalles de temps réguliers une pression transmembranaire de référence, correspondant par exemple à la pression transmembranaire du filtre calculée à sa première mise en service pour un débit de liquide de dialyse fixé, à la pression transmembranaire instantanée mesurée au même débit, et lorsque celle-ci s'écarte de celle-là d'une quantité prédéterminée, elle émet une alarme ou un message sur une unité d'affichage (non représentée) pour signaler à l'utilisateur que le filtre 21 doit être changé.

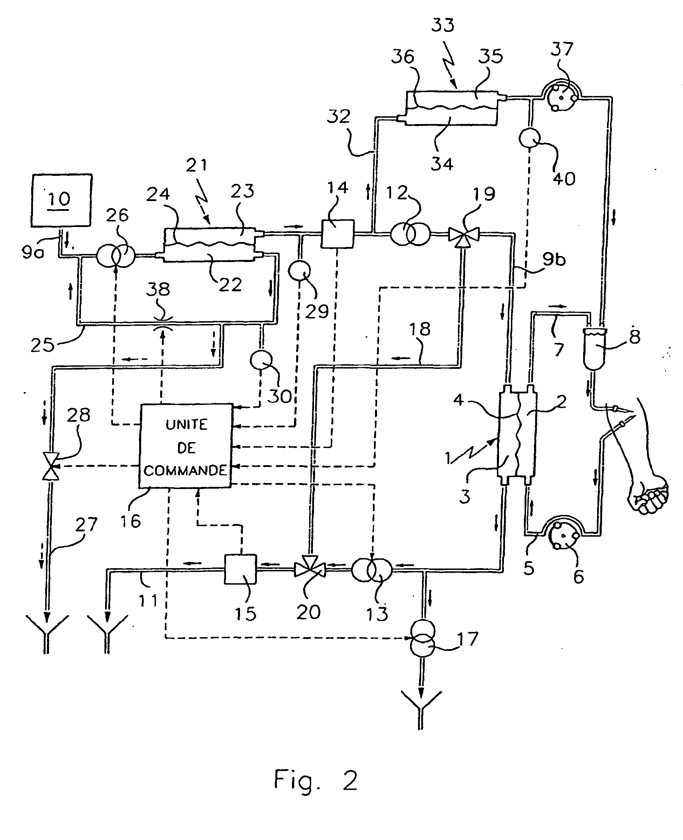

La figure 2 représente un dispositif d'hémodiafiltration qui se différencie du dispositif d'hémodialyse décrit ci-dessus par les caractéristiques suivantes (les composants de ces deux dispositifs remplissant les mêmes fonctions ont été désignés par les mêmes chiffres de référence).

Conformément à sa vocation, ce dispositif comporte des moyens pour infuser au patient un liquide de substitution destiné à compenser, généralement en partie, la quantité d'eau plasmatique soutirée du circuit vasculaire du patient par ultrafiltration dans l'hémodialyseur à haute perméabilité 1. Les moyens d'infusion comprennent une canalisation de liquide de substitution 32 ayant une première portion reliant la canalisation d'alimentation de liquide de dialyse 9a, 9b à une entrée d'une première chambre 34 d'un second filtre 33, et une seconde portion reliant une sortie d'une seconde chambre 35, séparée de la première chambre 34 par une membrane de filtration 36, au piège à bulles 8 du circuit de circulation extracorporelle de sang. Une pompe de circulation 37 de liquide de substitution est disposée sur la seconde portion de la canalisation de liquide de substitution 32. La canalisation de liquide de substitution est connectée à la canalisation d'alimentation 9a, 9b entre l'organe pour mesurer une quantité de liquide de dialyse 14 (débitmètre par exemple) et la première pompe 12 de circulation de liquide de dialyse.

Dans ce dispositif, le premier filtre 21 n'est pas connecté à la canalisation d'alimentation 9a, 9b de liquide de dialyse à l'intérieur du système de maîtrise volumétrique d'ultrafiltration (débitmètres 14, 15, portion du circuit de liquide de dialyse s'étendant entre les débitmètres et pompe d'ultrafiltration 17) mais à l'extérieur de ce système, en amont du premier débitmètre 14. Comme cela a été mentionné plus haut, grâce à cette disposition le liquide circulant dans le premier débitmètre 14 est un liquide filtré, ce qui prévient l'encrassement et, le cas échéant, le blocage accidentel, de cet organe de mesure sensible.

Autre différence avec le dispositif représenté sur la figure 1, la pompe de balayage 26 est disposée immédiatement en amont de la première chambre 22 du premier filtre 21, sur une portion de canalisation commune à la canalisation d'alimentation 9a et à la canalisation de bouclage 25. En fonctionnement, la pompe de balayage 26 est réglée à un débit supérieur à la somme des débits de la pompe de circulation 12 et de la pompe de liquide de substitution 37, de façon qu'un débit de recirculation déterminé s'établisse dans la canalisation de bouclage 25. Un organe de rétreint 38, éventuellement réglable, est disposé sur la canalisation de bouclage 25, grâce auquel il est possible d'obtenir une pression suffisante dans le circuit de liquide de dialyse en amont de la pompe de circulation 12 pour que la pression dans la seconde chambre 35 du second filtre 33, telle que mesurée par un capteur de pression 40, soit toujours positive ou au minimum nulle, quel que soit le débit de la pompe de liquide de substitution 37. L'organe de rétreint 38 peut être constitué par une portion calibrée de la canalisation de bouclage 25.

Le fonctionnement de ce deuxième dispositif est le suivant, pour ce qu'il diffère de celui du dispositif représenté sur la figure 1.

Les diverses pompes de l'installation tournant aux débits prescrits par l'opérateur ou programmés par défaut ou calculés, soit en début soit en cours de séance, l'unité de calcul et de commande 16 compare, soit en continu, soit à intervalles de temps réguliers, la pression mesurée au moyen du capteur 29 dans la seconde chambre 23 du filtre 21 à une pression de consigne et elle pilote la pompe de balayage 26 de façon que la pression instantanée tende vers la pression de consigne. La pression de consigne est choisie pour que la pression dans la seconde chambre 35 du second filtre 33, telle que mesurée par le capteur de pression 40, soit toujours positive ou au minimum nulle, compte tenu du débit de liquide de substitution imposé à la pompe 37.

Pour mesurer le degré d'encrassement de la membrane du filtre 21, deux procédés sont possibles. Soit, comme décrit ci-dessus, la pression transmembranaire dans le filtre 21 est calculée grâce aux données fournies par les capteurs de pression 29, 30 et est comparée à une pression transmembranaire de référence mise en mémoire (par exempte, la pression transmembranaire mesurée en début de séance). Soit, la vitesse de rotation de la pompe de balayage 26 est comparée à une vitesse de référence (par exemple, la vitesse de rotation mesurée en début de séance et mise en mémoire dans l'unité de commande 16). Comme la pression est maintenue constante dans la deuxième chambre 23 du filtre 21 par le réglage de la vitesse de rotation de la pompe de balayage 26, si la membrane 24 s'encrasse, la vitesse de rotation de la pompe sera augmentée d'autant. Lorsque la valeur mesurée du paramètre considéré (pression transmembranaire ou vitesse de rotation de la pompe de balayage) atteint la valeur de référence correspondante, l'unité de commande 16 commande l'ouverture de la vanne de purge 28 le temps nécessaire à l'évacuation du liquide contenu dans la première chambre 23 du filtre 21 et dans la canalisation de bouclage 25.

Bien que cette variante puisse être considérée comme moins avantageuse, la canalisation de purge 27 du dispositif de la figure 1 pourrait être connectée à la canalisation d'évacuation 11 de liquide usé à l'intérieur du système de maîtrise volumétrique de l'ultrafiltration, c'est-à-dire en amont des seconds moyens de mesure 15 d'une quantité de liquide usé.

Par ailleurs, l'organe de contrôle de débit 28 disposé sur la canalisation de purge 27 pourrait être constitué par une pompe. Dans le dispositif de la figure 2, cette pompe pourrait tourner de façon continue de façon que les impuretés retenues par la membrane soient éliminées au fur et à mesure.

## Revendications

1. Procédé de détermination du vieillissement d'un filtre (21) ayant deux chambres (22, 23) séparées par une membrane de filtration (24), une première chambre (22) étant reliée à une source (10) de liquide de traitement et une seconde chambre (23) ayant une sortie pour du liquide de traitement filtré **caractérisé en ce qu'**il comprend les étapes de :
- mesurer et mémoriser, pour un débit de liquide de traitement donné à la première mise en service du filtre, une pression transmembranaire de référence dans le filtre (21);
- mesurer, à chaque utilisation ultérieure du filtre (21) ou à intervalles de temps réguliers, la pression transmembranaire au débit de liquide de traitement donné;
- comparer la pression transmembranaire mesurée à la pression transmembranaire de référence.

2. Procédé selon la revendication 1, **caractérisé en ce qu'**il comporte en outre étapes de :
- calculer, à partir de la pression transmembranaire de référence, une pression transmembranaire limite correspondant à une limite d'utilisation du filtre (21) ;
- émettre une alarme lorsque la pression transmembranaire mesurée, au débit de liquide de traitement donné, atteint la pression transmembranaire limite.

## Patentansprüche

1. Verfahren zur Bestimmung der Alterung eines Filters (21) mit zwei Kammern (22, 23), die durch eine Filtrationsmembran (24) voneinander getrennt sind, wobei eine erste Kammer (22) mit einer Behandlungsflüssigkeitsquelle (10) verbunden ist und eine zweite Kammer (23) einen Ausgang für filtrierte Behandlungsflüssigkeit besitzt, **dadurch gekennzeichnet, dass** es die Schritte umfasst:
- Messen und Speichern eines Bezugstransmembrandruckes im Filter (21) für eine bei der ersten Inbetriebnahme des Filters vorgegebene Behandlungsflüssigkeitsdurchflußrate;
- Messen des Transmembrandruckes bei der vorgegebenen Behandlungsflüssigkeitsdurchflußrate bei jeder weiteren Verwendung des Filters (21) oder in regelmäßigen Zeitintervallen;
- Vergleichen des gemessenen Transmembrandruckes mit dem Bezugstransmembrandruck.

2. Verfahren nach Anspruch 1, **dadurch gekennzeichnet, dass** es weiter die Schritte umfasst:
- Berechnen eines Grenztransmembrandruckes, der einer Verwendungsgrenze des Filters (21) entspricht, aus dem Bezugstransmembrandruck;
- Aussenden eines Alarms, wenn der gemessen Transmembrandruck bei der vorgegebenen Behandlungsflüssigkeitsdurchflußrate den Grenztransmembrandruck erreicht.

## Claims

1. A method for determining the ageing of a filter (21) having two compartments (22, 23) separated by a filtration membrane (24), a first compartment (22) being connected to a treatment liquid source (10) and a second compartment (23) having an outlet for filtered treatment liquid, **characterized in that** it includes the following steps:
- measuring and storing, for a given treatment liquid flow rate at first commissioning of the filter, a reference transmembrane pressure in the filter (21);
- measuring, at every further use of the filter (21) or at regular time intervals, the transmembrane pressure at the given treatment liquid flow rate;
- comparing the measured transmembrane pressure with the reference transmembrane pressure.

2. The method according to claim 1, **characterized in that** it further comprises the following steps:
- calculating, starting from the reference transmembrane pressure, a limit transmembrane pressure corresponding to a use limit of the filter (21);
- sending out an alarm when the measured transmembrane pressure, at the given treatment liquid flow rate, reaches the limit transmembrane pressure.
